(19) Europäisches Patentamt
European Patent Office
Office européen des brevets

(11) Numéro de publication: **0 136 938**
**A2**

(12) **DEMANDE DE BREVET EUROPEEN**

(21) Numéro de dépôt: 84401763.2

(22) Date de dépôt: 05.09.84

(51) Int. Cl.[4]: C 07 H 15/14
C 07 H 15/26, C 07 H 15/18
C 07 H 15/04, C 07 D 319/12
C 07 H 15/24, C 07 D 307/60
C 07 D 265/30, C 07 D 407/12
A 61 K 31/70, A 61 K 31/34
//(C07D407/12, 319:00, 311:00)

(30) Priorité: 05.09.83 FR 8314179

(43) Date de publication de la demande: 10.04.85 Bulletin 85/15

(84) Etats contractants désignés: AT BE CH DE GB IT LI LU NL SE

(71) Demandeur: Etablissement Public dit: CENTRE NATIONAL DE LA RECHERCHE SCIENTIFIQUE (CNRS)
15, Quai Anatole France
F-75007 Paris(FR)

(72) Inventeur: Monsigny, Michel
341, rue des Bouvreuils
F-45590 Saint-Cyr-En-Val(FR)

(72) Inventeur: Delmotte, Francis
150, rue Philippe de Commynes
F-45160 Olivet(FR)

(72) Inventeur: Roche, Annie-Claude
360, route de Saint-Mesmin
F-45750 Saint-Pryve(FR)

(74) Mandataire: Nony, Michel
Cabinet Nony 29, rue Cambacérès
F-75008 Paris(FR)

(54) **Nouveaux dérivés furaniques, leur préparation et leur application.**

(57) Nouveaux dérivés de formule générale I:

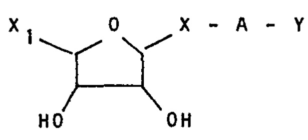

(I)

dans laquelle:

$X_1$ représente un atome d'hydrogène, un groupement alkyle ou un groupement hydroxyalkyle;

X représente un groupement –O–, –S–, –SO–, $-SO_2-$, $-N(R_1)-$ ou une liaison covalente directe entre le cycle furanique et le groupement –A–Y–, $R_1$ étant un atome d'hydrogène ou un groupement alkyle;

A représente un groupement divalent hydrocarboné ayant 1 à 8, notamment 1 à 6 atomes de carbone, et pouvant être interrompu par un ou plusieurs hétéroatomes ou groupements d'hétéroatomes et/ou par un ou plusieurs groupements tels que –CO–NH–; ou bien –X–A– représente une liaison covalente directe entre le cycle furanique et le groupement Y;

Y représente un groupement réactif $Y_1$ permettant la fixation par couplage du dérivé de formule I sur un groupement réactif autre qu'un alcool d'une molécule ou macromolécure ($M_1$) susceptible de constituer un médicament, un réactif biologique, un réactif en chromatographie d'affinité, ou une matrice pouvant servir de support de chromatographie ou de réactif biologique, ou

Y représente $-Y_2-M_1$, $Y_2$ représentant le groupement résultant de la transformation du groupement fonctionnel $Y_1$ après couplage avec la molécule ($M_1$), et $M_1$ représentant le reste dérivé de la molécule ($M_1$) après couplage.

Application notamment à la fixation de molécules ou macromolécules d'origine biologique sur des supports spécifiques, y comprise des supports de chromatographie.

Croydon Printing Company Ltd

0136938

Nouveaux dérivés furaniques, leur préparation et leur application

La présente invention a pour objet de nouveaux dérivés furaniques, leur préparation et leur application, notamment leur fixation sur des supports spécifiques.

Ces nouveaux dérivés bifonctionnels permettent la fixation, en deux étapes successives et indépendantes, de deux molécules aminées ou d'une molécule phénolique et d'une molécule aminée. Autrement dit, les nouveaux dérivés bifonctionnels de la présente demande servent d'agents de couplage pour la fixation d'une molécule aminée ou phénolique sur une autre molécule aminée.

Dans le domaine de la chromatographie et notamment de la chromatographie d'affinité, on a déjà proposé de fixer des molécules, ayant les propriétés d'affinité souhaitées, à des matrices (généralement des polymères) par l'intermédiaire de composés bifonctionnels ou agents de couplage dont le caractère bifonctionnel permet d'établir des liaisons, en particulier des liaisons covalentes, d'une part avec la molécule douée de propriétés d'affinité et d'autre part avec le support solide. Outre leur fonction de fixation de la molécule désirée sur le support, les agents de couplage peuvent remplir en outre diverses fonctions, et notamment une fonction d'éloignement de ladite molécule de la surface du support afin d'éliminer ou de diminuer les éventuels effets propres du support.

On a également proposé de relier des médicaments, par l'intermédiaire de tels agents de couplage, à des supports spécifiques tels que des hormones, des anticorps, etc... de façon à transporter ledit médicament spécifiquement sur le récepteur intéressé.

En particulier, dans le domaine de la lutte contre le cancer, on a cherché à coupler les médicaments anti-cancéreux à des supports spécifiques afin que lesdits médicaments détruisent spécifiquement les cellules cancéreuses sans nuire aux cellules saines pour lesquelles lesdits médicaments sont généralement fortement toxiques. Une revue exposant l'objet de ces recherches et les résultats auxquels elles ont abouti a été effectuée notamment par T.GHOSE et A.J. BLAIR, (ANTIBODY-LINKED CYTOTOXIC AGENTS IN THE TREATMENT OF CANCER : CURRENT STATUS AND FUTURE PROSPECTS), J. NATL CANCER INST, volume 61, n°5, Septembre 1978 pages 657 à 676.

La présente invention a notamment pour objet de nouveaux dérivés furaniques, utilisables notamment comme agents de couplage bifonctionnels, ainsi que les dérivés furaniques monofonctionnels correspondants obtenus après un premier couplage. Ces derniers dérivés sont utilisables par exemple dans le domaine de la chromatographie d'affinité ou dans le domaine thérapeutique pour la fixation de médicaments ou de réactifs biologiques sur des supports spécifiques. L'invention a plus précisément pour objet les nouveaux dérivés de formule générale I :

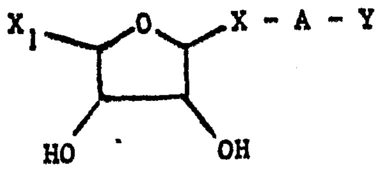

(I)

dans laquelle :

$X_1$ représente un atome d'hydrogène, un groupement alkyle ou un groupement hydroxyalkyle ;

X représente un groupement -O-, -S-, -SO-, $-SO_2-$, $-N(R_1)-$ ou une liaison covalente directe, entre le cycle furanique et le groupement -A-Y, $R_1$ étant un atome d'hydrogène ou un groupement alkyle ;

A représente un groupement divalent hydrocarboné ayant 1 à 8, notamment 1 à 6 atomes de carbone,et pouvant être interrompu par un ou plusieurs hétéroatomes ou groupements d'hétéroatomes et/ou par un ou plusieurs groupements tels que -CO-NH- ; ou bien -X-A- représente une liaison covalente directe entre le cycle furanique et le groupement Y ;

Y représente un groupement réactif $Y_1$ permettant la fixation par couplage du dérivé de formule I sur un groupement réactif autre qu'un alcool d'une molécul ou macromolécule ($M_1$) susceptible de constituer un médicament, un réactif biologique, un réactif en chromatographie d'affinité, ou une matrice pouvant servir de support de chromatographie ou de réactif biologique,

ou Y représente $-Y_2-M_1$, $Y_2$ représentant le groupement résultant de la transformation du groupement fonctionnel $Y_1$ après couplage avec la molécule ($M_1$), et $M_1$ représentant le reste dérivé de la molécule ($M_1$) après couplage.

L'invention a en particulier pour objet les dérivés de formule I pour lesquels Y (= $Y_1$) représente -CO-R' , $-NH_2$ , $-NO_2$ ,

-Ar-R''', 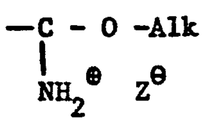 ou 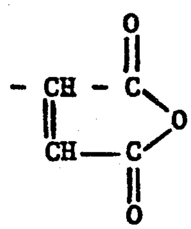

R' représentant un groupement $-O^{\ominus}M^{\oplus}$ , -OR" ou 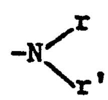

R" représentant un atome d'hydrogène, un groupement alkyle ou aralkyle ou un groupement succinimidyle,

r et r' représentant indépendamment un atome d'hydrogène ou un groupement alkyle inférieur ou bien représentant ensemble, avec l'atome d'azote, un hétérocycle à 5 ou 6 chaînons,

Ar représentant un groupement arylène,

R''' représentant un groupement $-NH_2$ , $-NO_2$ , -N=C=S, ou $-N{=}N^{\oplus}$ $Z^{\ominus}$ ,

et $Z^{\ominus}$ étant un anion.

L'invention a en particulier pour objet les dérivés de formule I dans lesquels le cycle furanique a la configuration du D-ribose, du L-ribose, du L-lyxose et du D-lyxose. Autrement dit l'invention a notamment pour objet les dérivés du D-ribose de formule Ia :

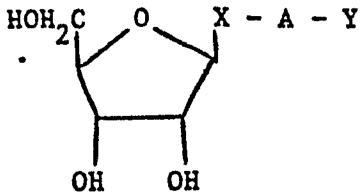

(Ia)

les dérivés du L-ribose de formule Ib :

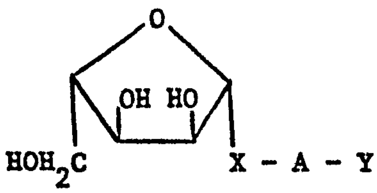

(Ib)

les dérivés du D-lyxose de formule Ic :

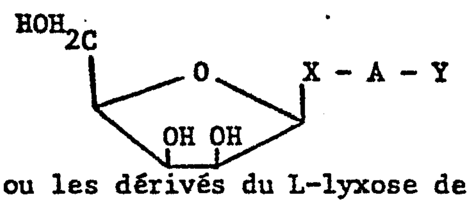

(Ic)

ou les dérivés du L-lyxose de formule Id :

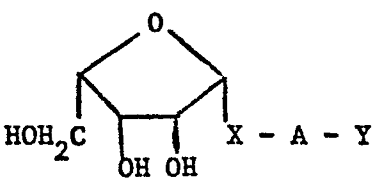

(Id)

Dans la formule I, les substituants alkyle ou hydroxyalkyle sont notamment des groupements alkyle inférieur ou hydroxyalkyle inférieur qui ont par exemple de 1 à 8 atomes de carbone ; $X_1$ représente notamment un hydroxyméthyle; A représente notamment un groupement alkylène ayant 1 à 8 atomes de carbone pouvant être interrompu par un ou plusieurs hétéroatomes ou groupements d'hétéroatomes tels que -NH- et/ou par un ou plusieurs groupements tels que -CO-NH ; par exemple A représente un groupement $-(CH_2)_m-NH-(CH_2)_m-$ , m étant un nombre entier pouvant varier de 1 à 4, ou A représente un groupement

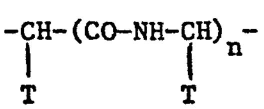 ,

n étant un nombre entier pouvant varier de 0 à 6 et T étant la chaîne latérale d'un acide α-aminé ; en outre A peut être relié à Y par l'intermédiaire d'un groupement hétéroatomique terminal tel que par exemple le groupement -S-S- ; lorsque R" représente un groupement alkyle il s'agit notamment d'un groupement alkyle ayant 1 à 3 atomes de carbone ; lorsque R" représente un groupement aralkyle il s'agit notamment d'un groupement benzyle éventuellement substitué, tel qu'un groupement nitrobenzyle ou aminobenzyle ; lorsque r et r' représentent,

avec l'atome d'azote auquel ils sont reliés un reste hétérocyclique à 6 chaînons, il s'agit notamment d'un reste de triazine éventuellement substituée par un ou plusieurs halogènes, par exemple un reste de dichlorotriazine ; le groupement Ar représente notamment un groupement phénylène éventuellement substitué et pouvant comporter 1 à 3 hétéroatomes d'azote ; en particulier Ar peut représenter un groupement ortho- , méta- ou para-phénylène, ou un groupement de formule

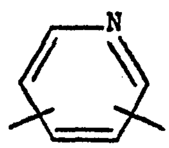

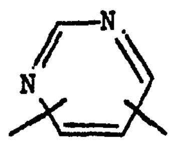

ou

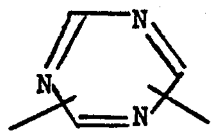

$Z^{\ominus}$ représente notamment un anion minéral tel qu'un anion halogénure, ou un anion organique tel qu'un anion d'acide carboxylique ; $M^{\oplus}$ représente notamment un proton ou un cation métallique, par exemple un cation de métal alcalin ou alcalino-terreux.

Parmi les composés de formule I on citera en particulier ceux pour lesquels le groupement -X-A-Y (égal à $-X-A-Y_1$) représente l'un des groupements suivants :

a) $-S-CH_2-CO_2H$ et les dérivés correspondants :

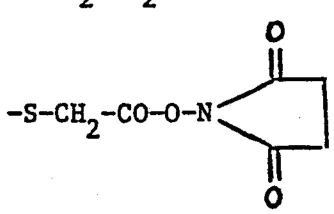

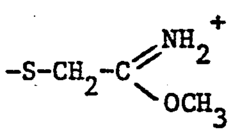

$Z^{\ominus}$

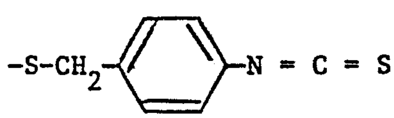

$-S-CH_2-CO_2-CH_3$

$-S-CH_2-CO-NH-R$,

R étant un reste de protéine tel qu'un reste de lectine ou un reste de lectine succinylée, un reste d'enzyme tel qu'un reste de peroxydase, un reste de drogue aminée tel qu'un reste de daunorubicine, un reste de puromycine, un reste de primaquine ou un reste de chloroquine, un reste d'adriamycine, un reste de bléomycine, un reste d'aminoacide, ou un reste de peptide;

$-S-CH_2-CO-NH-Fl$ avec Fl représentant:

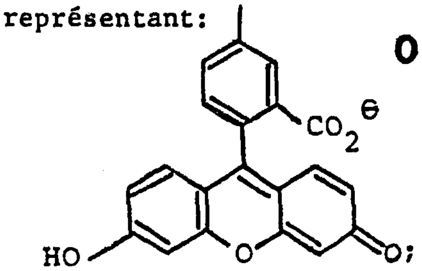

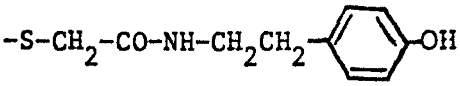

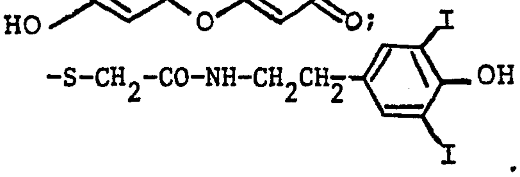

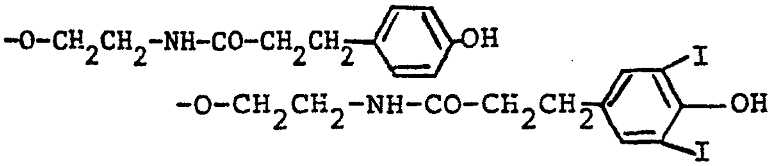

b) les groupements de formules :

$-O-CH_2-CH_2-NH_2$

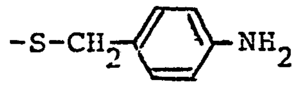

c) 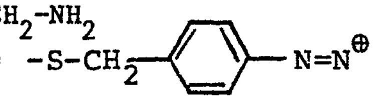 $Z^{\ominus}$

d) $-O-CH_2-CH_2-NH-CO-CH_2-I$

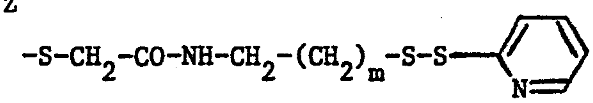

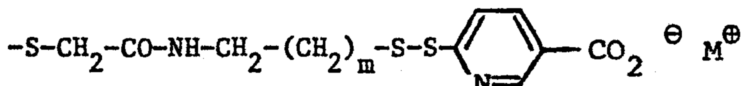

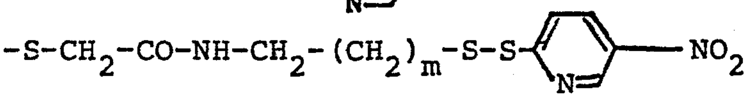

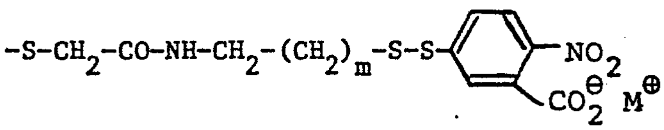

m étant défini comme précédemment ;

e) 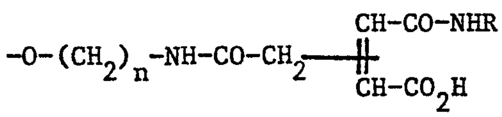

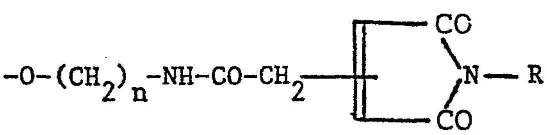

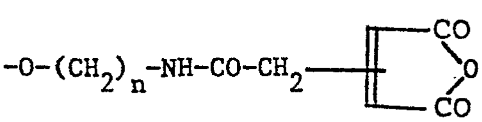

R étant défini comme précédemment et n étant un nombre entier variant de 1 à 6 ;

f) 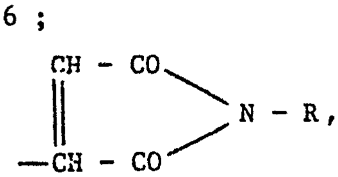 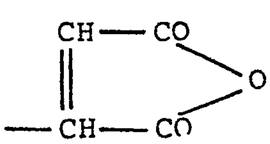 ou 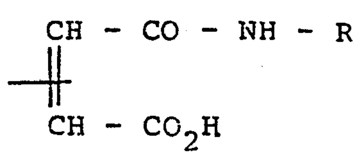

R étant défini comme précédemment.

L'invention a également pour objet les composés de formule I décrits ci-après dans la partie expérimentale.

Les composés de formule I (avec $Y = Y_1$) peuvent être préparés selon les méthodes usuelles de la chimie organique, et notamment selon les méthodes décrites ci-après dans la partie expérimentale ou selon des méthodes analogues.

Comme indiqué ci-dessus, le groupement $-Y$ ($= -Y_1$) dans la formule I est choisi de façon à pouvoir permettre la fixation du dérivé de formule I sur des molécules ou macromolécules très diverses, en particulier par l'établissement d'une liaison de covalence par réaction du groupement $Y_1$ avec un groupement réactionnel, autre qu'un alcool, desdites molécules ou macromolécules. Par exemple lorsque $-X-A-Y_1$ représente un groupement tel que donné au a), e) ou f) du paragraphe précédent, il est possible de fixer le dérivé I sur une molécule contenant un groupement aminé ;
lorsque $X-A-Y_1$ représente un groupement tel que donné au b) du paragraphe précédent, il permet de fixer le dérivé I sur une molécule contenant un groupement carboxylique ; lorsque $-X-A-Y_1$ représente un groupement tel que celui donné au c) du paragraphe précédent, il permet de fixer le dérivé I sur une molécule comportant un groupement aromatique, notamment un groupement aromatique activé par une fonction phénol ou amine, par réaction de formation d'un dérivé azoïque ou sur une molécule comportant un groupement aminé comme dans la lysine, un groupement guanidine comme dans l'arginine ou un groupement imidazole comme dans l'histidine ; lorsque $-X-A-Y_1$ représente un groupement tel que ceux indiqués au d) du paragraphe précédent, cela permet de fixer le dérivé I sur une molécule contenant un groupement thiol.

Il convient de remarquer que les groupements réactifs $Y_1$ sont tels qu'ils permettent la réaction des composés de formule I (avec $Y = Y_1$) sur les molécules ($M_1$) dans des conditions (solvant, température, pH) compatibles avec le respect de la structure ou de l'activité des molécules ($M_1$) qui sont essentiellement des molécules d'origine biologique.

Bien entendu, les molécules ou macromolécules ($M_1$) et les groupements $Y_1$ des composés de formule I sont choisis de façon que $Y_1$ ne réagisse pas avec les groupements alcools que les molécules ($M_1$) possèdent éventuellement, car dans ce cas la réaction serait concurrencée par la réaction des composés I entre eux, c'est-à-dire par réaction du groupement $Y_1$ sur les groupements OH du cycle furanose.

On obtient donc ainsi, par la fixation sur une molécule ($M_1$), d'un composé de formule I (avec $Y=Y_1$), un nouveau dérivé de formule II :

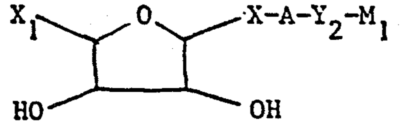

(II)

dans laquelle $X_1$ , X et A sont définis comme dans la formule I, $Y_2$ représente l groupement résultant de la transformation du groupement fonctionnel $Y_1$ après couplage avec la molécule ($M_1$), et $M_1$ représente le reste dérivé de la molécul ($M_1$) après couplage. On voit que la formule II correspond à la formule I avec $Y = -Y_2-M_1$.

Parmi les molécules ($M_1$) utilisables pour obtenir les dérivés de

formule II on citera notamment des antibiotiques, en particulier des antibiotiques toxiques vis-à-vis des cellules cancéreuses, comme par exemple l'Adriamycine, la Daunomycine (ou Daunorubicine), la Bléomycine ; des peptides tels que par exemple les peptides suivants :

-Arg-Leu
-Ala-Ala-Pro-Ala
-Ala-Ala-Pro-Val
-Gly-Gly-Gly
-Phé-Leu-Phé
-Ala-Leu-Ala-Leu
-Tyr-Leu
-Tyr-Gly-Glu-Phé-Leu
-Gly-Pro-Arg
-Phé-Pro-Arg
-Arg-Arg, etc. ;

des antimétabolites tels que la puromycine, l'acide phosphonacétylaspartique, etc. ; des toxines telles que par exemple les lectines, les lectines succinylées, la toxine du ricin, la chaîne A de la toxine du ricin, la gélonine, la chaîne A de l'abrine, de la modeccine, de la toxine diphtérique ou de la toxine tétanique ; des enzymes telles que des phospholipases, des peroxydases, des phosphatases, des glycosidases, des protéases, etc. ; des dérivés fluorescents tels que la fluorescéine, l'amino-rhodamine, le rouge de Texas, etc. ; des molécules susceptibles d'être couplées à des agents traceurs radioactifs, comme par exemple la tyramine, la thyroxine, etc. ; des molécules ou macromolécules aminées pouvant servir de site réactif en chromatographie d'affinité biospécifique, chimique ou physico-chimique : il s'agit en particulier de molécules susceptibles de donner lieu à des interactions réversibles de type biospécifique, chimique ou physico-chimique, par exemple des interactions ioniques, des interactions faisant intervenir des propriétés d'hydrophobie, des interactions faisant intervenir une affinité biospécifique ou chimique, par exemple la formation de complexes réversibles; lesdites molécules ou macromolécules aminées peuvent avoir un caractère cationique susceptible d'être mis à profit dans des réactions d'échange d'anions ; elles peuvent comporter des groupements anioniques tels que les groupements carboxylique ou sulfonique et donner lieu à des réactions d'échange de cations ; une molécule ou macromolécule aminée peut servir de site réactif en chromatographie biospécifique et constituer par exemple un substrat ou un inhibiteur d'enzymes, un récepteur d'hormones, de protéines, de virus ou de bactéries, un antigène ou un anticorps. Ladite molécule ou macromolécule aminée ($M_1$) peut être notamment une globuline, notamment une gammaglobuline, une toxine bactérienne, un antigène bactérien, un antigène

viral, un ganglioside ou un dérivé de ganglioside, la lysine, la taurine, la phénylalanine, la phénylhydrazine, etc... Lorsque $-Y_1$ comporte un groupement $-NH_2$, la molécule $(M_1)$ est notamment un agent acylant dérivé du groupement que l'on veut fixer tel que par exemple un ester d'hydroxysuccinimide. On notera enfin que la molécule $(M_1)$ peut être constituée par une molécule active couplée à un peptide qui servira d'intermédiaire de fixation de $(M_1)$, au dérivé de formule I, et qui permettra en outre, dans certains cas, la libération in situ de la molécule active.

L'intérêt des produits de formule II réside en particulier dans le fait qu'il est possible de rendre réactif le groupement glycol du cycle furanique par la réaction connue de coupure oxydante des glycols vicinaux, qui conduit à la formation d'un dialdéhyde pouvant réagir avec des molécules ou macromolécules aminées $(M_2)$ pour donner, après réduction de la base de Schiff résultante, un dérivé de formule V

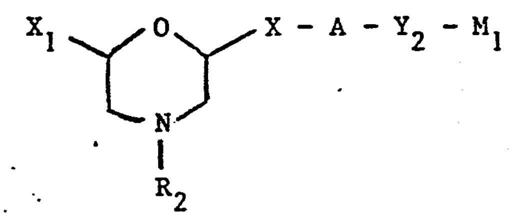

(V)

dans laquelle X, $X_1$, A, $Y_2$ et $M_1$ sont définis comme dans la formule II et $R_2$ représente le reste de la molécule aminée $(M_2)$ lorsque celle-ci est écrite sous la forme $R_2-NH_2$.

La réaction de coupure oxydante des glycols vicinaux à l'aide de l'acide periodique est bien connue et a été décrite en détail notamment par Ernest L. JACKSON, ORGANIC REACTIONS volume II, pages 341-375 (JOHN WILEY & SONS, 7ème édition, 1960). On sait que cette réaction de coupure oxydante peut également être effectuée à l'aide d'autres agents oxydants, et notamment le tétracétate de plomb.

La réduction de la base de Schiff (qui est facultative comme expliqué ci-après) est effectuée à l'aide d'un agent réducteur tel qu'un hydrure, par exemple un borohydrure alcalin.

Les molécules $(M_2)$ sont des molécules ou macromolécules aminées telles que celles mentionnées ci-dessus dans la définition de $(M_1)$, par exemple des anticorps ou des fragments d'anticorps F(ab) ou $F(ab')_2$, et en particulier des anticorps spécifiques obtenus soit par hyperimmunisation selon les méthodes connues à l'aide d'antigènes hautement purifiés, soit par chromatographie d'affinité, soit encore par production d'anticorps monoclonaux, lesdits anticorps monoclonaux pouvant être préparés selon la méthode générale décrite par KENNETH et al. "Monoclonal antibodies, Hybridomas : New dimension of biological analysis", Plenum Press, New York, London 1980, pages 3 à 30".

Parmi les molécules ($M_2$) on citera également des toxines (par exemple des lectines), et aussi les polymères aminés tels que l'aminoéthylamino-dextran, l'aminoéthylamino-cellulose ou l'aminoéthylamino-amidon ; l'agarose (tel que l'Ultrogel A2, A4 ou A6) ou le Sépharose 2B, 4B ou 6B substitué par exemple par l'hexaméthylène diamine ; les dérivés aminés du polytrisacryl (en particulier les Ultrogèl GF, les Ultrogel AcA) etc.

Lesdites molécules ($M_2$) peuvent en outre être fixées sur un support solide par tous moyens connus, par exemple par absorption, par établissement d'une liaison covalente ou non, par exemple par réaction de couplage ou par affinité immunochimique.

Le support solide peut être par exemple un matériau solide biologique (globules rouges) ou synthétique doué de propriétés absorbantes ou capable de fixer un agent de couplage. Par exemple parmi les matériaux solides capables de fixer les anticorps par adsorption on citera notamment le polystyrène, le polypropylène, les latex.

Les agents de couplage permettant de fixer par covalence les molécules ($M_2$) sur un support solide sont également connus ; ce sont par exemple des dérivés bifonctionnels tels que des dialdéhydes (glutaraldéhyde) des di-isocyanates (toluène-di-isocyanate), des quinones (benzoquinone), etc... Les polymères aminés ($M_2$) peuvent également être fixés selon les méthodes connues sur des supports minéraux poreux tels que des supports de silice, d'alumine, de magnésie, de verre, de silicate, etc... Par couplage des produits de formule II avec des molécules ($M_2$) elles-mêmes fixées sur des supports solides, on peut obtenir notamment soit des supports utilisables en chromatographie, soit des réactifs biologiques.

Par exemple quand ($M_1$) représente un anticorps ou un antigène (éventuellement couplé à un agent traceur), et que ($M_2$) est un polymère constituant un support solide ou fixé à un support solide, on obtient un produit qui est susceptible de constituer un réactif de détection des anticorps ou des antigènes selon les méthodes classiques telles que la méthode "sandwich" ou la méthode dite de "compétition". Les agents traceurs sont par exemple des traceurs radioactifs obtenus par échange isotopique avec un isotope radioactif tel que $^{125}I$. Le couplage des anticorps ou des antigènes avec un agent traceur radioactif est connu en soi et peut être effectué par exemple selon la méthode décrite par HUNTER W.M. AND GREENWOOD, F.C., 1962, Nature, 194, 495.

L'agent traceur peut être également un traceur enzymatique couplé à l'anticorps ou à l'antigène selon une méthode analogue à celle décrite par AVRAMEAS, Immunochemistry, 6, 43-52 (1969).

Parmi les composés de formule I, on citera notamment:

0136938

- β-D-ribofuranyl-thio-1-éthanoate de méthyle;
- acide β-D-ribofuranyl-thio-1-éthanoïque;
- β-D-ribofuranyl thio-1-éthanoate d'hydroxysuccinimide;
- 1-p-nitrobenzylthio-β-D-ribofuranide;
- 1-p-aminobenzylthio-β-D-ribofuranide;
- p-isothiocyanate de β-D-ribofuranyl-thiobenzyle;
- 1-p-diazobenzylthio- β-D-ribofuranide;
- aminoéthyl-β-D-ribofuranoside;
- β-ribofuranylthio-éthanoyl-aminoéthyl-4-phénol;
- 4-β-ribofuranylthioéthanoyl aminoéthyl-2,6-di-iodophénol;
- O-(4-hydroxyphényl-propionamido-éthyl) β-D-ribofuranoside;
- O(3,5-diido-4-hydroxyphényl) propionamido-éthyl-β-D-ribofuranoside;
- ribofuranyl-thio-éthanoyl-fluorescéine; - ribofuranyl-thio-éthanoyl phénylalanine; - ribofuranyl-thio-éthanoyl daunomycine;
- ribofuranyl-thio-éthanoyl puromycine;
- le produit de conjugaison de l'acide ribofuranyl-thio-éthanoïque et d'une peroxydase, d'une lectine ou d'une lectine succinylée;
- le produit de formule

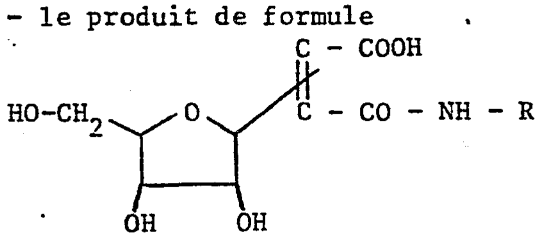

R étant défini comme précédemment;
- le produit de formule

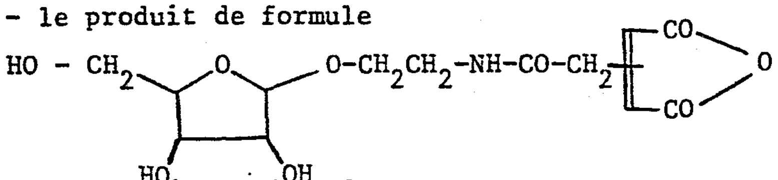

- le produit de formule

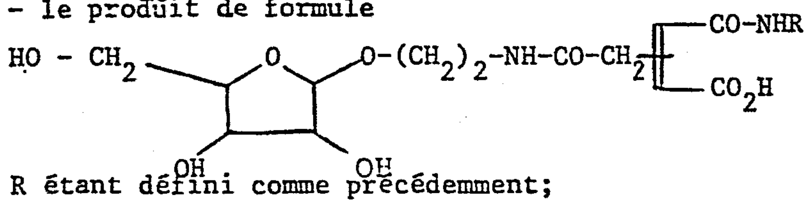

R étant défini comme précédemment;
- l'anhydride C-ribofuranylmaléique.

L'invention a également pour objet l'application des produits de formule I.

Cette application consiste à faire réagir, dans le cas où $Y = Y_1$, comme indiqué précédemment, le dérivé de formule I avec une molécule ($M_1$) capable de réagir avec le groupement réactif -Y pour former un dérivé de formule II, puis à soumettre, si désiré, le dérivé de formule II obtenu à une réaction de coupure oxydante pour former un dialdéhyde de formule III ou IIIA :

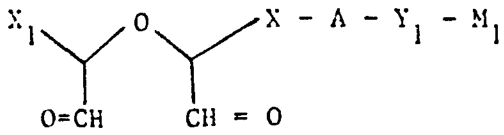

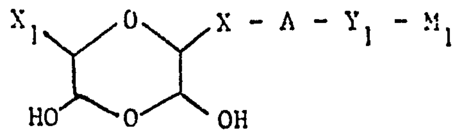

(III) ou (IIIA) (forme dihydrate)

que l'on fait réagir le dérivé III ou IIIA avec une molécule aminée ($M_2$) que l'on peut écrire sous la forme $R_2$-$NH_2$, telle que définie précédemment pour obtenir un dérivé de formule IV :

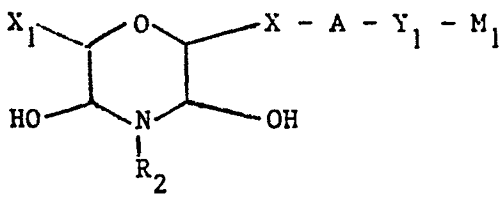

(IV)

$R_2$ étant le reste de ladite molécule ($M_2$) lorsqu'elle est écrite sous la forme $R_2$ $NH_2$, et que l'on soumet éventuellement le dérivé de formule IV à l'action d'un agent réducteur approprié pour obtenir le dérivé réduit correspondant ayant la formule (V) donnée ci-dessus.

Les composés V sont suffisamment stables pour pouvoir être utilisés directement, sans réduction en composé III.

La réduction du composé V est donc facultative.

Comme exemple d'application, dans le cas où Y = ($Y_1$) représente le reste d'anhydride maléique

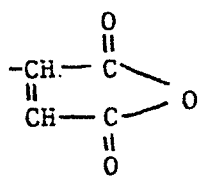

on peut faire réagir le produit de formule I avec une molécule ou macromolécule aminée ($M_1$ = $R_1NH_2$) pour obtenir un composé de formule

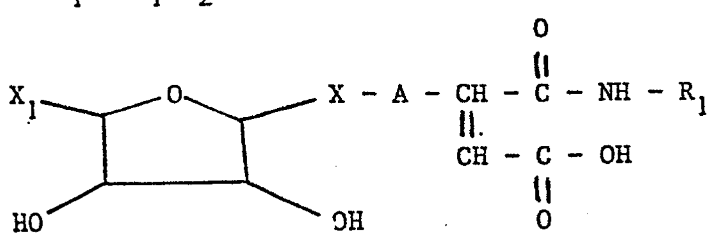

qui par oxydation periodique conduite au composé

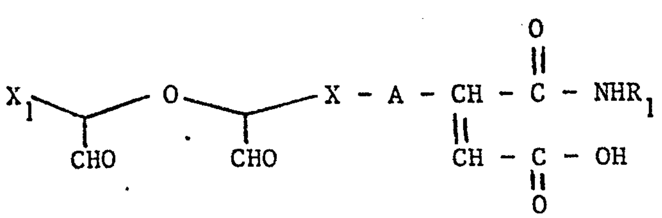

lequel, par réaction avec une molécule ou macromolécule aminée ($M_2$ = $R_2NH_2$) fournit le composé de formule

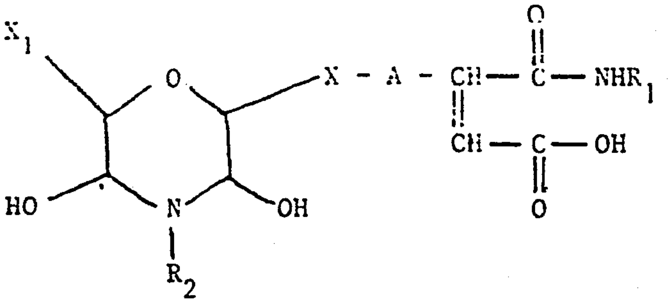

L'intérêt de ce dernier composé ou du composé réduit correspondant

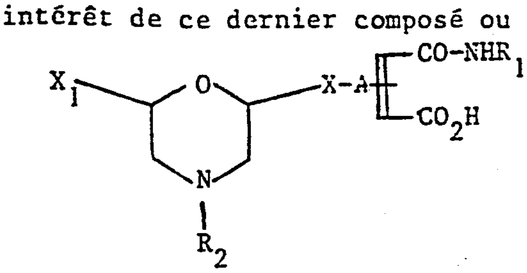

est qu'il libère facilement le composé $R_1NH_2$ par hydrolyse en milieu légèrement acide. L'invention a également pour objet les produits III ou IIIA, IV et V.

Les exemples suivants illustrent l'invention sans toutefois la limiter.

Exemple 1

Préparation du β-D-ribofuranyl thioéthanoate d'hydroxysuccinimide.

Stade 1 : le 1-O-acétyl-2,3,5-tri-O-benzoyl-β-D-ribofuranose (produit commercialisé par les firmes Aldrich, Serva et Pfanstiehl) (10g) est dissous dans l'éther éthylique anhydre (350ml) saturé en gaz chlorhydrique à 0°C. Le ballon réactionnel est abandonné 5 jours à 4°C, à l'abri de l'humidité. Le solvant est évaporé sous pression réduite. Le résidu est dissous dans l'acétone anhydre (400ml).

A la solution obtenue de 1-chloro-2,3,5-tri-O-benzoyl-α-D-ribofuranose on ajoute de la thiourée (1,7g), puis on porte à reflux pendant 1 heure.

On obtient le chlorure de 1-isothiouronium-2,3,5-tri-α-benzoyl-β-D-ribofuranose qui cristallise au cours du refroidissement.

Rendement 79 %; p.f: 176-177°C ; $[\alpha]^{24}_{589}$ : -48°.

Stade 2 : ledit chlorure (2g) est mis en suspension dans l'acétone (120ml) en présence de méthylbromoacétate (0,5ml), puis dissous par addition d'une solution aqueuse (60ml) de dithionite de sodium (1,25g) en présence de carbonate de potassium (0,99g). Le mélange réactionnel est agité à 25°C pendant 2 heures, puis est évaporé à siccité. Le résidu est extrait au chloroforme (3x20ml), la phase organique est lavée à l'eau, séchée sur sulfate de sodium anhydre, puis concentrée à siccité. Le sirop obtenu est cristallisé dans l'éthanol.

On obtient ainsi le méthyl ester du tri-O-benzoyl-2,3,5-β-D-ribofu-

ranyl-thio-1-éthanoate.

Rendement 73 %; p.f. 74°C ; $\alpha_{589}^{27}$ = -52° ($CHCl_3$).

Stade 3 : le composé obtenu au stade 2 est quantitativement débenzoylé par le méthanolate de sodium dans un mélange dichlorométhane/méthanol (1:3,v/v) à 4°C pendant 5 heures. Après neutralisation par la résine polystyrène sulfonée AG 50W-X8 forme $H^+$ dans le méthanol, le mélange réactionnel est filtré, puis extrait à l'eau. La phase aqueuse après évaporation au méthanol est lyophilisée. On obtient ainsi un sirop incolore correspondant au méthyl ester du β-D-ribofuranyl-thio-1-éthanoate.

Rendement : 90 %.

Stade 4 : le composé obtenu au stade 3 est saponifié par une solution aqueuse de soude, puis neutralisé par une résine AG 50W-X8 forme $H^+$. La solution aqueuse est lyophilisée, en donnant un sirop incolore correspondant à l'acide β-D-ribofuranyl-thio-1-éthanoïque.

Rendement : 96 %.

Stade 5 : le composé obtenu au stade 4 est dissous dans le N-diméthylformamide en présence d'hydroxysuccinimide. En maintenant la température à 0°C, on additionne le dicyclohexyl-carbodiimide et la solution est agitée pendant 16 H. Après filtration de la dicyclohexylurée, le filtrat est évaporé à siccité. On obtient le β-D-ribofuranyl-thio-1-éthanoate d'hydroxysuccinimide.

Rendement : 95 %

Exemple 2

Préparation du p-isothiocyanate de β-D-ribofuranyl-thio-benzyle.

Stade 1 : le composé obtenu au stade 1 (5g) de l'exemple 1 est mis en suspension dans l'acétone (300ml) contenant du bromo p-nitrobenzyle (2,2g), On ajoute une solution réductrice aqueuse (150ml) contenant du dithionite de sodium (3,1g) et du carbonate de potassium (2,5g). La solution obtenue est agitée 2 heures à 25°C. L'acétone est évaporée. On extrait le résidu par le chloroforme. La solution chloroformique est lavée à l'eau, puis séchée sur sulfate de sodium.

La phase organique est concentrée en sirop. On obtient le 1-p-nitrobenzylthio-2,3,5-O-tribenzoyl-β-D-ribofuranide.

Rendement : 95 %.

Stade 2 : le composé obtenu au stade 1 (4,3g) est débenzoylé par le méthanolate de sodium 1M (7ml) en présence de dichlorométhane (60ml) et de méthanol anhydre (60ml) à température ambiante durant 1 heure. Le mélange réactionnel est évaporé sous pression réduite ; le sirop obtenu est chromatographié sur une colonne de silice dans le système solvant chloroforme/méthanol (9:2,v/v). On obtient le 1-p-nitrobenzylthio-β-D-ribofuranide qui cristallise par dissolution dans le méthanol et le chloroforme (v/v) suivie d'addition d'hexane.

Rendement : 50 % ; p.f. 95-96°C

Stade 3 : le composé du stade 2 dissous dans le méthanol est soumis à une réduction par l'hydrogène sur charbon palladié en présence d'un équivalent d'acide chlorhydrique pendant 5 heures à 25°C. Après filtration, le solvant est éliminé par évaporation sous pression réduite. Le résidu repris dans un minimum de méthanol et chloroforme (v/v), cristallise par addition d'hexane. On obtient le 1-p-aminobenzylthio-β-aminobenzylthio-β-D-ribofuranide.

Rendement : 50 %. p.f. 117-118°C.

Stade 4 : le composé du stade 3 est dissous dans une solution de bicarbonate de sodium 0,1M. On additionne sous agitation vigoureuse un volume équivalent de thiophosgène à 0,1 % dans le chloroforme. L'agitation est maintenue 20 minutes. Le mélange réactionnel est décanté. La phase aqueuse est recueillie puis lavée au chloroforme. Après concentration de la phase aqueuse le p-isothiocyanate de β-D-ribofuranyl-thiobenzyle cristallise.

Rendement : 90 %

Exemple 3

Préparation du 1-p-diazobenzylthio-β-D-ribofuranide.

A une solution (6ml) du composé du stade 3 de l'exemple 2 (0,2m mole) contenant de l'acide chlorhydrique (0,5 m mole) et du bromure de potassium (0,04 m mole) refroidie à 0-2°C, on additionne goutte à goutte une solution (2ml) de nitrite de sodium (0,3 m mole). Le mélange réactionnel est agité une heure à 0°C.L'excès d'acide nitreux est éliminé par addition durée et agitation 30 minutes. On obtient une solution de chlorhydrate de 1-p-diazobenzylthio-β-D-ribofuranide.

Exemple 4

Préparation de l'aminoéthyl-β-D-ribofuranoside.

Stade 1 : à une solution de 1-O-acétyl-2,3,5-tri-O-benzoyl-β-D-ribofuranose (1g) dans du dichlorométhane anhydre (40ml) refroidie à 0°C, on additionne du chlorure stannique (0,24ml). A cette solution amenée à température ambiante on additionne le nitroéthanol (0,22ml). La réaction est complète au bout de 30 minutes. Le mélange réactionnel est versé goutte à goutte dans 50ml d'une solution glacée de carbonate de sodium. On extrait par la dichlorométhane. L'extrait est lavé à l'eau puis séché au sulfate de sodium et évaporé à sec sous pression réduite. On obtient le 1-O-β-D-nitroéthyl-2,3,5-trio-O-benzoyl-β-D-ribofuranoside.

Rendement : 92 %.

Stade 2 : le composé obtenu au stade 1 (535mg) est mis en solution dans le méthanol (40ml)en présence de charbon palladié (500mg) et d'un équivalent d'acide chlorhydrique. Le mélange réactionnel est placé sous pression (1atm, soit $1,01.10^5$ Pa) d'hydrogène pendant 8 heures. Après filtration et évaporation, le chl

rhydrate du 1-O-aminoéthyl-2,3,5-tri-O-benzoyl-β-D-ribofuranoside cristallise dans un système solvant méthanol/chloroforme/hexane.

Rendement : 68 %. p.f. 117-118°C.

Stade 3 : Par action du méthanolate de sodium sur le composé du stade 2, comme décrit dans l'exemple 1, stade 3, on obtient le 1-O-aminoéthyl-β-D-ribofuranoside qui cristallise dans un mélange solvant méthanol/chloroforme (1:5,v/v).

Rendement : 81 %.

**Exemple 5**

Préparation du β-ribofuranyl thioéthanoyl aminoéthyl-2,6-di-iodophénol. Couplage sur une protéine.

Stade 1 : au composé de l'exemple 1 stade 5 (75mg) dissous dans 2ml de diméthylformamide, on additionne à 0°C une solution 30ml de tyramine (l'équivalent). La réaction est totale en 15 heures. Le solvant est évaporé à sec, le sirop obtenu est purifié par chromatographie sur silice dans le système solvant chloroforme/méthanol (9:2,v/v).

On obtient le β-ribofuranyl thioéthanoylaminoéthyl phénol.

Stade 2 : le composé de stade 1 est iodé, dans les conditions classiques par action de l'iodure de sodium en présence de chloramine T. On obtient le β-ribofuranylthioéthanoyl-aminoéthyl-2,6 diiodophenol. Lorsque l'iodure de sodium utilisé est radioactif ($^{123}I$, $^{125}I$ ou $^{131}I$) on obtient un réactif radioactif qui remplace avantageusement le réactif de BOLTON et HUNTER (Biochem J. 1973, 133, 529), 3-(p-hydroxy phényl) propionate de succinimidyle.

Stade 3 : le composé du stade 2 est dissous dans l'acétate de sodium 0,2M pH5,0 contenant une quantité équivalente de periodate de sodium. La solution est maintenue 20 minutes à 0°C. Dans ces conditions on obtient quantitativement l'hydrate de dialdéhyde de formule :

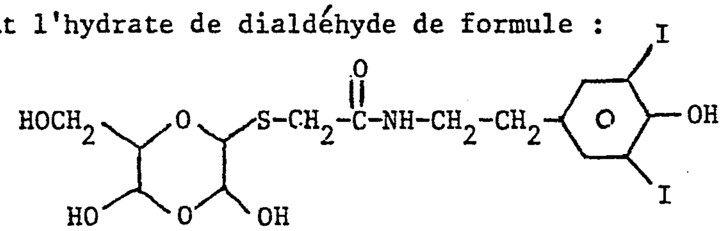

Stade 4 : On fait réagir le composé du stade 3 en solution aqueuse avec une protéine (lectine du germe de blé dans un tampon carbonate-bicarbonate de sodium 0,4 M de pH 9,5). La solution est maintenue à 25°C pendant 4 heures. On ajoute une quantité de $BH_4$ Na équivalente à la quantité de periodate de sodium et la solution est maintenue 1h à 25°C.

La protéine subtituée obtenue est purifiée par chromatographie de tamisage moléculaire (Ultrogel GF 05, 2cmx40cm) dans un tampon tris HCl 0.5 M, NaCl 0,1 M, pH 7,5. Elle est substituée par 10 molécules de di-iodo tyramine par molécule.

Cette protéine substituée est parfaitement stable en solution aqueuse.

Exemple 6

Préparation du O-(3,5-di-iodo-4-hydroxyphényl-propionamido-éthyl)-β-D-ribofuranoside. Couplage sur une protéine.

Stade 1 : La réaction du composé de l'exemple 4 stade 3 avec le 3-(4-hydroxyphényl) propionate de succinimidyle est conduite dans les conditions décrites dans l'exemple 5 stade 1. On obtient le O-(4-hydroxyphényl propionamido-éthyl)-β-D-ribofuranoside.

Stade 2 : Le composé du stade 1 est iodé, dans les conditions classiques, par action de l'iodure de sodium en présence de chloramine T. On obtient le O-(3,5 di-iodo-4-hydroxyphényl propionamido-éthyl)-β-D-ribofuranoside. Lorsque l'iodure de sodium utilisé est radioactif ($^{123}I$, $^{125}I$, ou $^{131}I$) on obtient un réactif radioactif qui remplace avantageusement le réactif de BOLTON et HUNTER.

Stade 3 : on oxyde le composé du stade 2, par le periodate dans les conditions décrites à l'exemple précédent. Le composé oxydé de formule :

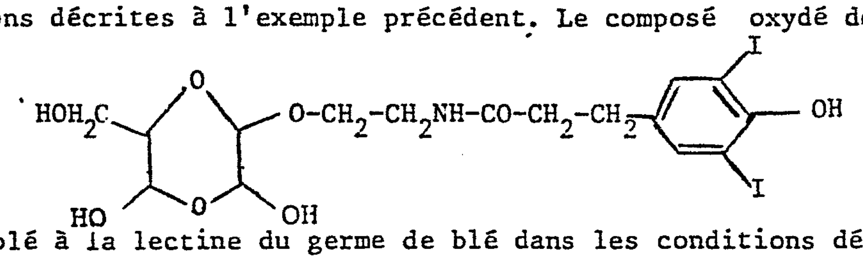

est couplé à la lectine du germe de blé dans les conditions décrites à l'exemple précédent. La protéine substituée par le dérivé iodé de l'hydroxyphényl-propionoyle est purifiée par chromatographie de tamisage moléculaire sur Ultrogel GF05. La protéine ainsi obtenue est substituée par 10 molécules du dérivé iodé de l'hydroxyphénylpropionoyle par molécule de protéine.

Cette protéine substituée est parfaitement stable en solution aqueuse.

Exemple 7

Préparation du ribofuranyl-thio-éthanoylamino-fluorescéine.

Le composé de l'exemple 1 stade 3 (1 millimole) est dissous dans 2ml de N-diméthylformamide. A cette solution refroidie à 4°C, on ajoute sous atmosphère d'azote un équivalent d'aminofluorescéine, un équivalent d'hydroxybenzotriazole, un équivalent de dicyclohexyl carbodiimide et un équivalent de triéthylamine. Le mélange réactionnel est maintenu 2 heures à 0°C puis 18 heures à 25°C. La dicyclohexylurée précipite en maintenant le mélange réactionnel à 0°C pendant 18 heures, puis est éliminée par filtration. Le N-diméthyl formamide est éliminé par évaporation sous pression réduite. Le résidu est purifié par chromatographie sur colonne de silice dans le système solvant $CHCl_3/CH_3OH/H_2O$ (80:30:3).

0136938

Les fractions contenant le composé de formule :

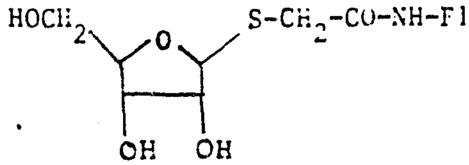

où Fl représente le radical

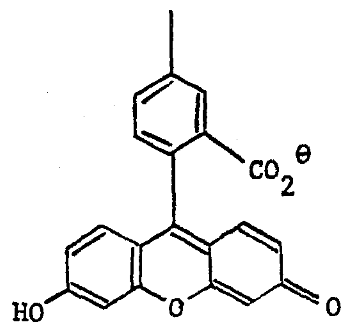

sont évaporées sous pression réduite.

Le résidu obtenu est repris dans un tampon acétate de sodium puis oxydé par le periodate de sodium dans les conditions décrites à l'exemple 5. Le dialdéhyde obtenu, de formule :

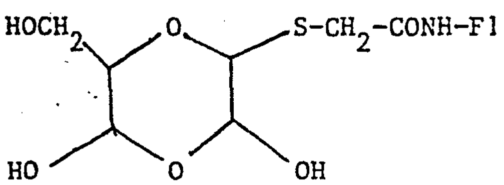

réagit sur une protéine (la lectine du germe de blé) dans les conditions décrites à l'exemple 5. Dans ces conditions, on peut fixer 3 molécules de fluorescéine par molécule de lectine.

<u>Exemple 8</u>

Insolubilisation de la lectine du germe de blé.

Le ribofuranyl thioéthanoate de succinimidyle est dissous dans le diméthylformamide à 0°C.

Cette solution est ajoutée à une solution de lectine du germe de blé dans un tampon NaCl 0.1 M-borate de sodium 0,1M, pH 8,5. La solution est agitée à 4°C pendant 24 heures. La protéine est ensuite purifiée par chromatographie sur Ultrogel GF05 dans un tampon acétate de sodium saturé. La lectine ribosylée est ensuite succinylée par l'anhydride succinique.

La lectine ribosylée et succinylée est ensuite traitée au periodate de sodium dans les conditions décrites à l'exemple 5, stade 3. La lectine succinylée et oxydée (50mg) est mise en contact avec l'Ultrogel A4 (5ml) substitué par l'hexaméthylène diamine à 4°C dans un tampon carbonate-bicarbonate de sodium, pH 9,5. La suspension est agitée pendant 18 heures à 4°C.

A cette solution on ajoute le borohydrure de sodium (1mg/mg), et la suspension est agitée 1 heure à 4°C. Le gel est ensuite lavé dans un tampon NaCl 0,1M, tris HCl 0,05 M pH 7,5. Dans ces conditions, le gel est substitué par 10mg

de lectine par ml. On rappelle que l'Ultrogel A4 est constitué par une matrice d'agarose à 4 %.

Exemple 9

Insolubilisation de la D-phénylalanine.

Le composé de l'exemple 1 stade 5 réagit avec la D-phénylalanine dans des conditions analogues à celle décrite dans l'exemple 5 stade 1, mais en présence d'un équivalent de triéthylamine.

Le composé ainsi obtenu est soumis à l'oxydation périodique dans les conditions décrites à l'exemple 5 stade 3. Le composé oxydé de formule :

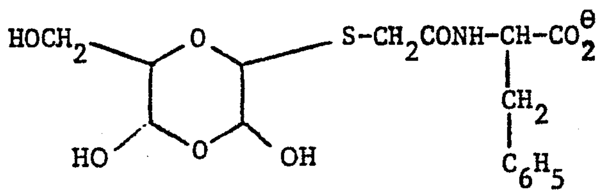

est mis en présence d'Ultrogel A4 substitué par l'hexaméthylène diamine, dans un tampon carbonate-bicarbonate de sodium pH 9,5. Après 18 heures d'agitation à 4°C, on ajoute du borohydrure de sodium (5mg/ml). La suspension est maintenue sous agitation à 4°C pendant 4 heures. Le gel est ensuite lavé par un tampon.

Dans ces conditions, 2mg de D-phénylalanine par ml de gel sont fixés. Cette colonne permet la purification de la carboxypeptidase par chromatographie d'affinité.

Exemple 10

Conjugué lectine-peroxydase

Le ribofuranyl thioéthanoate de succinimidyle est dissous dans le N-diméthyl formamide à 0°C. Cette solution est ajoutée à une solution de peroxydase de raifort dans un tampon borate de sodium 0,05 NaCl 0.1 M pH 8,5. La solution est agitée à 4°C pendant 18 heures. La protéine est ensuite purifiée par chromatographie de tamisage moléculaire sur Ultrogel GF05 dans un tampon acétate de sodium 0,2 M pH 5,0. Le dérivé β-D-ribofuranylthioéthanoyl-protéine correspondant est traité par un équivalent de periodate de sodium dans les conditions décrites à l'exemple 5 stade 3. Le produit oxydé correspondant (5mg) est ajouté à une solution de lectine du germe de blé (2mg) dans un tampon carbonate-bicarbonate de sodium pH 9,5. La suspension est agitée pendant 18 heures à 4°C. A cette solution, on ajoute le borohydrure de sodium (1mg/ml) et la suspension est agitée 1 heure à 4°C. Le complexe peroxydase-lectine est purifié par chromatographie de tamisage moléculaire sur colonne d'Ultrogel AcI34.
Dans ces conditions, on obtient le complexe peroxydase-lectine contenant 2 moles de peroxydase par mole de lectine avec un rendement de 60%.

Exemple 11

Dérivé de la daunorubicine

Le composé obtenu à l'exemple 1 stade 5 (0,15m mole) est dissous dans le N-diméthylformamide (2ml). A cette solution on ajoute du chlorhydrate de daunorubicine (0,15m mole) dissous dans le N-diméthylformamide en présence d'un équivalent de triéthylamine. La solution est agitée à 4°C pendant 16 heures. Le produit obtenu est purifié par chromatographie sur silice avec le système solvant :

$CHCl_3/CH_3OH/H_2O$ (80:30:3).

On obtient le produit de formule :

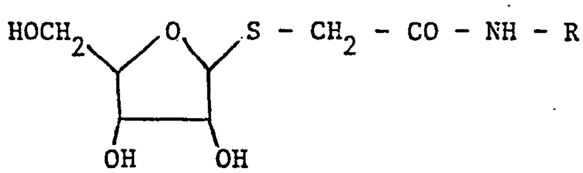

dans laquelle R-NH représente :

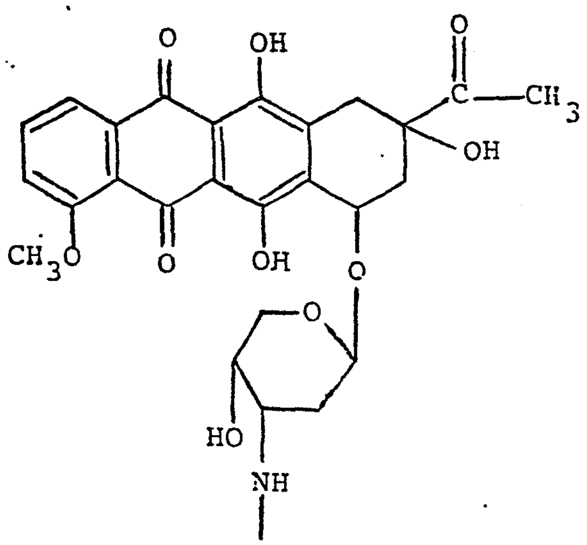

Exemple 12

Dérivé de la puromycine

Dans les mêmes conditions qu'à l'exemple précédent le composé de l'exemple 1 est couplé à la puromycine pour donner un produit de même formule mais dans laquelle R représente

0136938

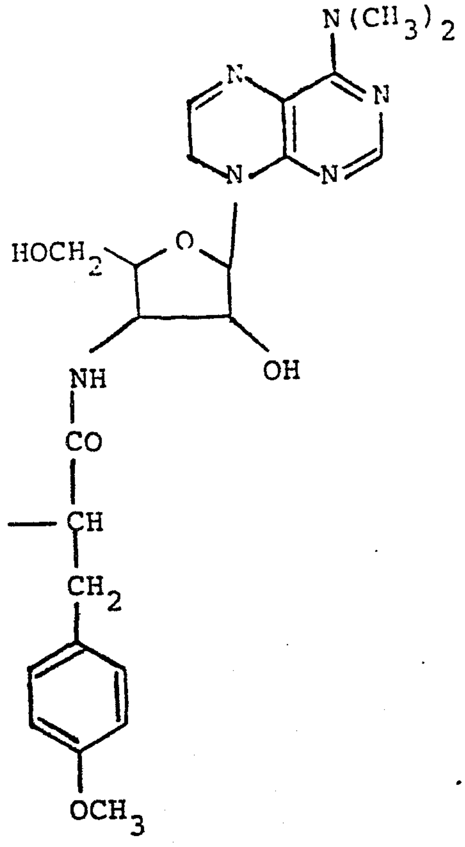

Exemple 13

Le composé de l'exemple 11 est dissous dans un volume de N-diméthylformamide à 0°C auquel on ajoute un volume d'une solution d'acétate de sodium 0,2 M, pH 5,0 contenant un équivalent de periodate de sodium. La solution est agitée à 0°C pendant 20 minutes. Cette solution est ajoutée dans une solution de lectine du germe de blé dans un tampon carbonate-bicarbonate de sodium 0,4 M, pH 9,5. La solution est maintenue à 25°C pendant 4 heures. A ce moment, est ajouté un équivalent de $NaBH_4$. La solution est agitée à 25°C pendant 1heure.

La protéine substituée obtenue est purifiée par chromatographie sur colonne de l'Ultrogel ACA 202. Le tampon utilisé est une solution aqueuse de NaCl 0.10 M et de tris-HCl 0.05 M de pH 7,5. La solution de protéine éluée de la colonne est dialysée contre de l'eau distillée, puis cette solution est lyophilisée.

De façon analogue on a préparé une lectine substituée par les produits des exemples 7 et 12.

Exemple 14 Anhydride C-ribofuranyl maléique

Ce produit est préparé selon le schéma suivant :

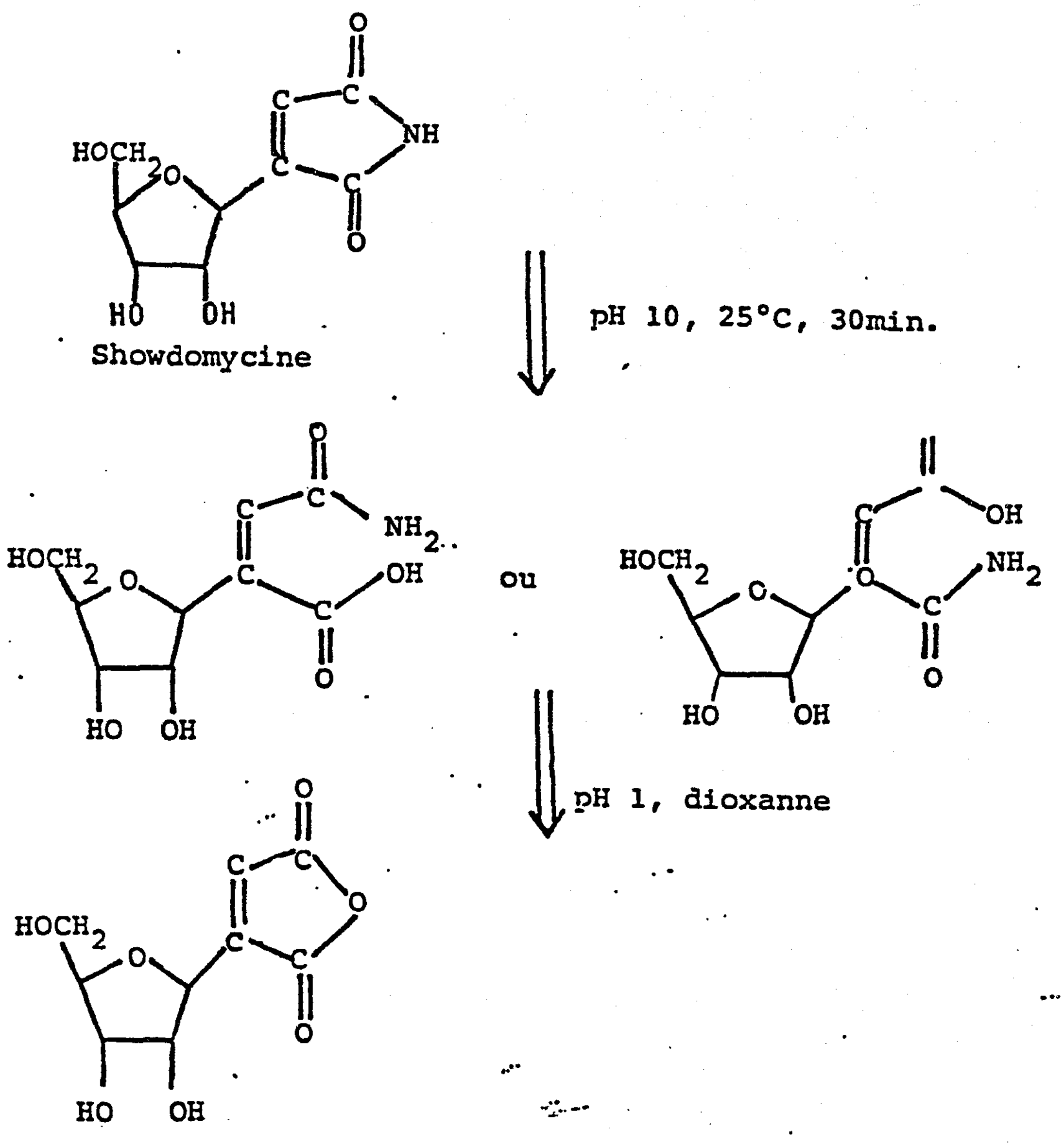


. La showdomycine 23mg (0,1mmole) est dissoute dans 1 ml de soude à 0,2M, à 25°C. Après 30min., la solution est neutralisée par addition de Dowex 50x8, ($H^+$, 20-50 mesh, soit 0,3-0,8mm) 500 mg. La suspension est lyophilisée, puis reprise dans le dioxane anhydre (5ml). Le dioxane est évaporé. Enfin, l'anhydride C-ribofuranyl maléique est dissous dans 2ml de N diméthylformamide (la résine Dowex 50 est éliminée par filtration). Cette solution peut être utilisée directement comme dans l'exemple 15. Dans les conditions décrites ci-dessus, la showdomycine est transformée quantitativement en anhydride C-ribofuranyl maléique.

Exemple 13

1 micromole d'anhydride C-ribofuranyl maléique est dissoute dans le N-diméthylformamide. A cette solution on ajoute 1 micromole de chlorhydrate de daunorubicine dissous dans le diméthylformamide et 2 équivalents de triéthanolamine (ou de N-éthyl morpholine). La solution est agitée à 25°C pendant 16 h. Le produit obtenu est purifié par chromatographie sur silice avec le système solvant : chloroforme/méthanol/eau 80 : 30 : 3 (en volume).

On obtient le produit conjugué correspondant de formule :

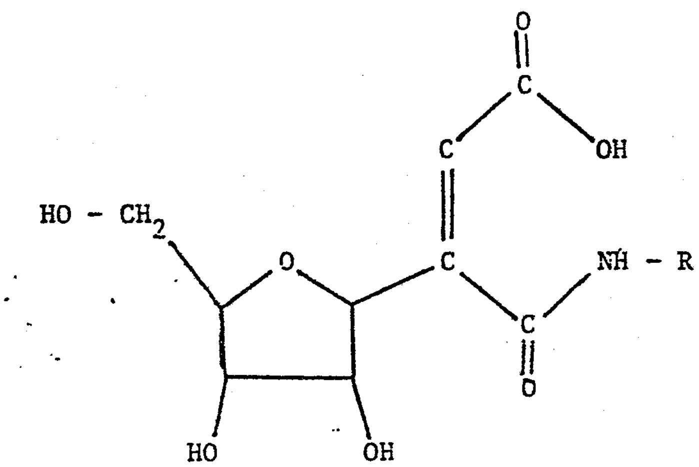

ou

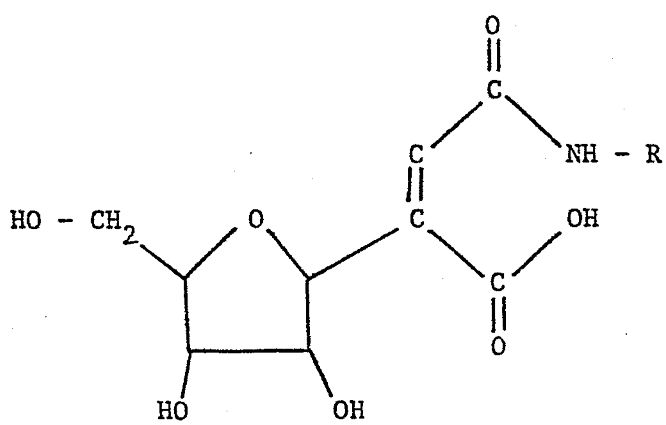

dans laquelle -NH-R représente le résidu daunorubicine montré dans l'exemple 11.

Le conjugué est dissous dans le N-diméthylformamide à 0°C auquel on ajoute un volume d'une solution de phosphate de sodium 0,2 M pH 7.2 contenant un équivalent de periodate de sodium. La solution est agitée à 0°C pendant 4 heures. Cette solution est ajoutée à une solution de 0,2 micromole de lectine de germe de blé dans un tampon carbonate-bicarbonate de sodium 0,4 M pH 9,5. La

solution est maintenue à 25°C pendant 4 heures puis traitée par le borohydrure comme à l'exemple 5. Le produit obtenu est purifié par chromatographie sur une colonne d'Ultragel ACA 202 dans un tampon phosphate de sodium 0,05 M, NaCl 0,10 M de pH 7.5. La solution est lyophilisée.

On obtient le produit suivant:

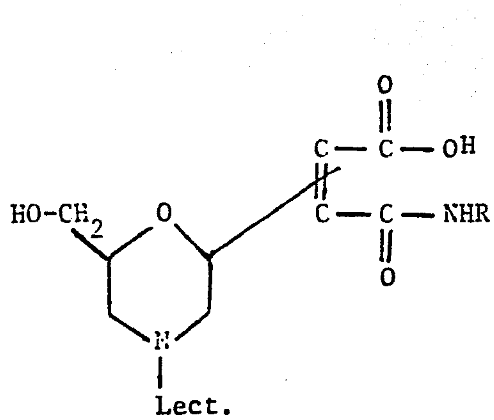

dans laquelle Lect -N< est le reste de la lectine de germe de blé Lect $-NH_2$ et -NH - R est le reste de la daunorubicine ($R-NH_2$).

La solution de protéine substituée par le conjugué oxydé est utilisée, après filtration sur Millipore 0,22 microns, dans des boîtes de culture contenant $10^6$ cellules leucémiques de souris L 1210. Après 4 heures, à 37°C, on effectue une extraction butanolique des cellules lysées par le Triton X 100, et l'on identifie la daunomycine libre par spectrofluorimétrie et chromatographie sur couche mince.

Dans une expérience de contrôle, on vérifie que la protéine substituée par le conjugué oxydé ne contient pas de daunomycine libre, à pH 7.5, mais qu'après traitement à pH 4.5 pendant 4 heures à 37°C, plus de 75 % de la daunomycine est libre.

Exemple 16

Anhydride ribofuranosyl éthylaminyl cis-aconitique.

L'anhydride cis-aconitiqe (156 mg, 1 mmole) est dissous dans 15 ml de N-diméthylformamide. On ajoute à cette solution à 25°C, l'aminoéthyl-β-D-ribofuranoside (exemple 4), (2m mole) dissous dans 10 ml de N-dimétnylformamide. Après 4 heures, la solution est refroidie à 4°C. On ajoute 2 m moles d'hydroxybenzotriazole et 2 mmoles de dicyclohexylcarbodiimide. Après 4 heures d'agitation, la solution est maintenue à 25°C pendant 20 heures, puis à 4°C pendant une nuit. Le précipité de dicyclohexylurée est éliminé par filtration.

Le composé

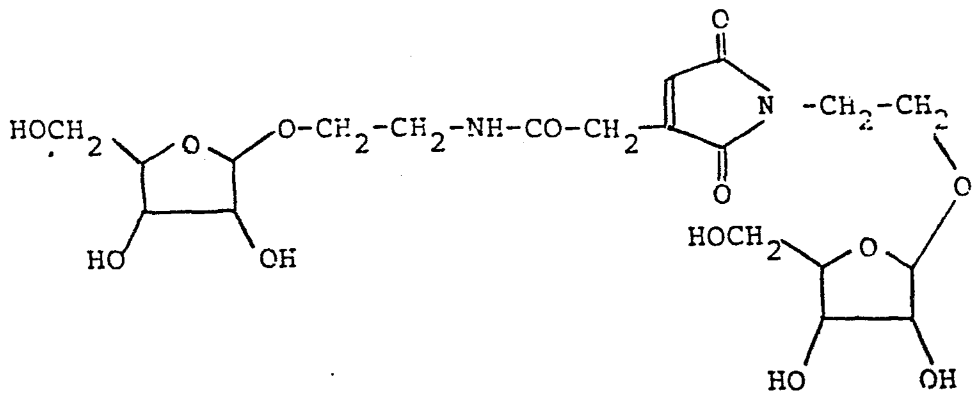

est purifié par chromatographie sur colonne de silice dans le mélange solvent chloroforme/méthanol 1:1 (v:v) puis après évaporation à siccité est traité comme la showdomycine.

On obtient le composé attendu :

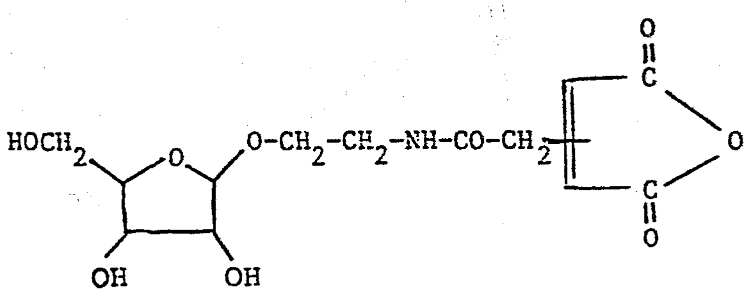

EXEMPLE 17

Le composé de l'exemple 16 réagit comme l'anhydride C-ribofuranylmaléique de l'exemple 15 c'est-à-dire que l'action de la daunorubicine donne le produit

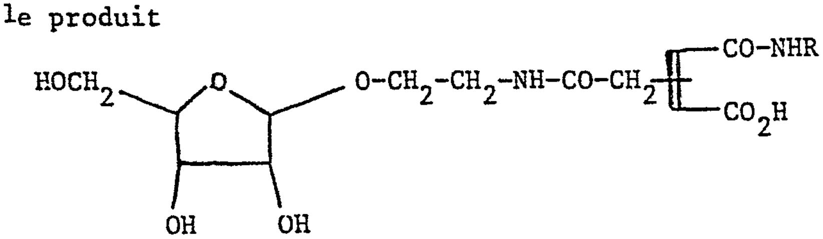

(-NHR étant le reste de la daunorubicine); l'oxydation periodique de ce produit donne le composé de formule:

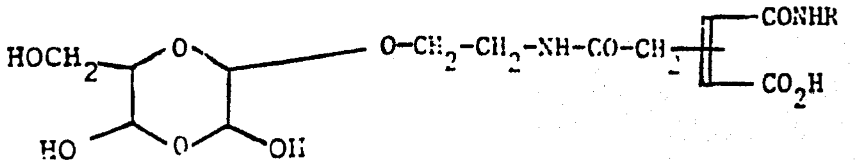

L'action de la lectine de germe de blé sur ce dernier produit donne le composé de formule

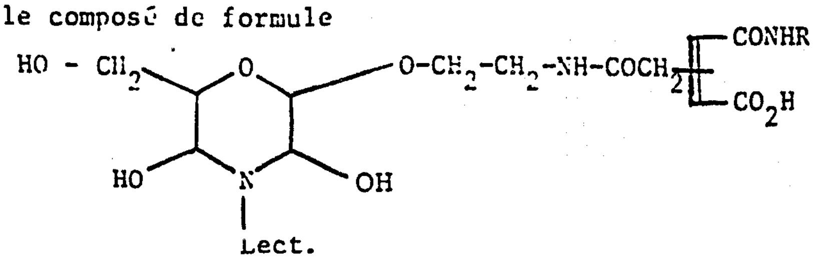

dont la réduction facultative par un borohydrure conduit au composé

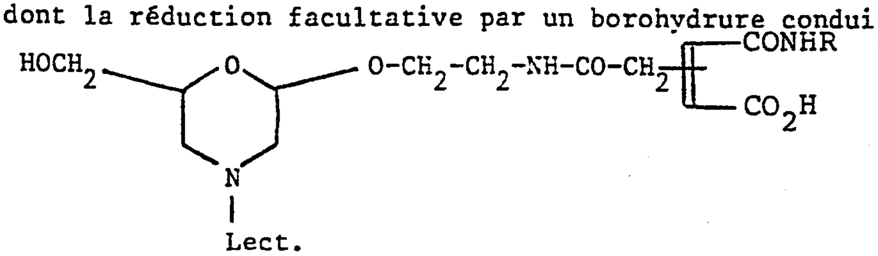

REVENDICATIONS

1. Nouveaux dérivés de formule générale I :

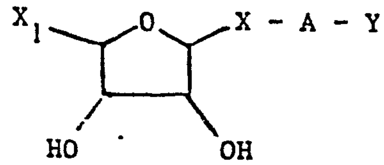

(I)

dans laquelle :

$X_1$ représente un atome d'hydrogène, un groupement alkyle ou un groupement hydroxyalkyle ;

X représente un groupement -O-, -S-, -SO-, $-SO_2-$, $-N(R_1)-$ ou une liaison covalente directe entre le cycle furanique et le groupement -A-Y-, $R_1$ étant un atome d'hydrogène ou un groupement alkyle ;

A représente un groupement divalent hydrocarboné ayant 1 à 8, notamment 1 à 6 atomes de carbone, et pouvant être interrompu par un ou plusieurs hétéroatomes ou groupements d'hétéroatomes et/ou par un ou plusieurs groupements tels que -CO-NH- ; ou bien -X-A- représente une liaison covalente directe entre le cycle furanique et le groupement Y ;

Y représente un groupement réactif $Y_1$ permettant la fixation par couplage du dérivé de formule I sur un groupement réactif autre qu'un alcool d'une molécule ou macromolécule ($M_1$) susceptible de constituer un médicament, un réactif biologique, un réactif en chromatographie d'affinité, ou une matrice pouvant servir de support de chromatographie ou de réactif biologique,

ou Y représente $-Y_2-M_1$, $Y_2$ représentant le groupement résultant de la transformation du groupement fonctionnel $Y_1$ après couplage avec la molécule ($M_1$), et $M_1$ représentant le reste dérivé de la molécule ($M_1$) après couplage.

2. Dérivés selon la revendication 1, caractérisés par le fait que Y ($=Y_1$) représente -CO-R', $-NH_2$, $-NO_2$,

-Ar-R"', 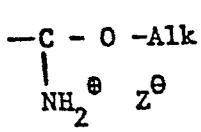 ou 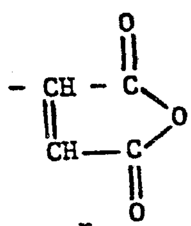

R' représentant un groupement $-O^{\ominus}M^{+}$, OR" ou 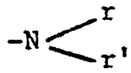 ,

R" représentant un atome d'hydrogène, un groupement alkyle ou aralkyle ou un groupement succinimidyle, r et r' représentant indépendamment un atome d'hydrogène ou un groupement alkyle inférieur ou bien représentant ensemble, avec l'atome d'azote, un hétérocycle à 5 ou 6 chaînons,

Ar représentant un groupement arylène,

R"' représentant un groupement $-NH_2$, $-NO_2$, -N=C=S, ou $-N{\equiv}N^{\oplus}$ $Z^{\ominus}$ , et $Z^{\ominus}$ étant un anion.

3. Dérivés selon l'une quelconque des revendications précédentes, caractérisés par le fait que le cycle furanique, dans la formule 1, a la configuration du D-ribose, du L-ribose, du L-lyxose ou du D-lyxose.

4. Dérivés selon l'une quelconque des revendications précédentes, caractérisés par le fait que A représente un groupement alkylène ayant 1 à 8 atomes de carbone, pouvant être interrompu par un ou plusieurs hétéroatomes ou groupements d'hétéroatomes tels que -NH- et/ou par un ou plusieurs groupements tels que -CO-NH-.

5. Dérivés selon la revendication 4, caractérisés par le fait que A représente un groupement $-(CH_2)_m-NH-(CH_2)_m-$, m étant un nombre entier pouvant varier de 1 à 4, ou A représente un groupement

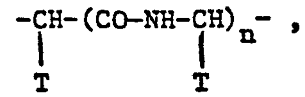
,

n étant un nombre entier pouvant varier de 0 à 6 et T étant la chaîne latérale d'un acide α-aminé, A pouvant en outre être relié à Y par l'intermédiaire d'un groupement hétéroatomique terminal tel que par exemple le groupement -S-S-.

6. Dérivés selon l'une quelconque des revendications précédentes, caractérisés par le fait que le groupement -X-A-Y représente l'un des groupements suivants :

a) $-S-CH_2-CO_2H$ et les dérivés correspondants :

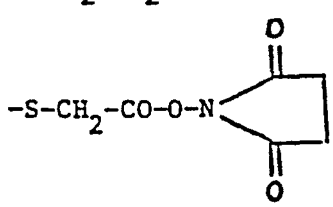

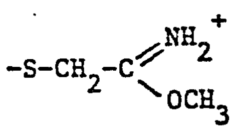
$Z^{\theta}$

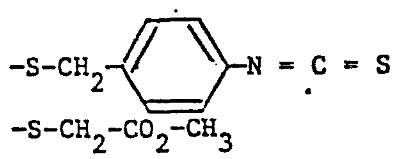

$-S-CH_2-CO_2-CH_3$

$-S-CH_2-CO-NH-R$,

R étant un reste de primaquine ou un reste de chloroquine, un reste d'adriamycine, un reste de bléomycine, un reste de lectine ou un reste de lectine succinylée, un reste d'enzyme tel qu'un reste de peroxydase, un reste de drogue aminée tel qu'un reste de daunorubicine, un reste de puromycine, un reste d'aminoacide, ou un reste de peptide ;

$-S-CH_2-CO-NH-Fl$ avec Fl représentant:

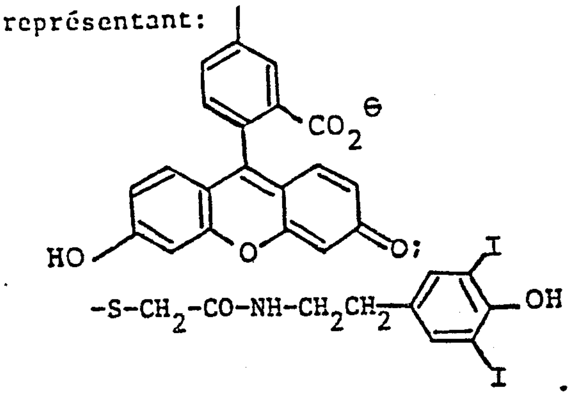

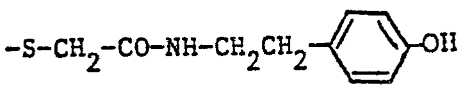

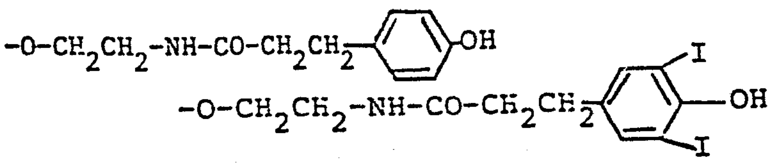

b) les groupements de formules :

$-O-CH_2-CH_2-NH_2$

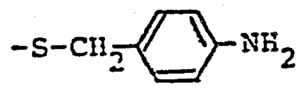

c) 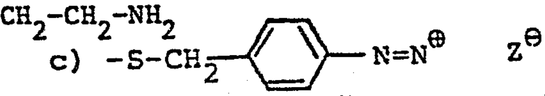

d) $-O-CH_2-CH_2-NH-CO-CH_2-I$

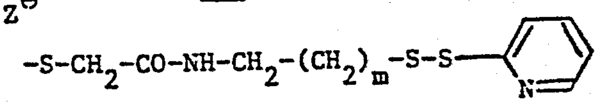

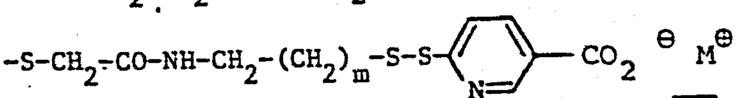

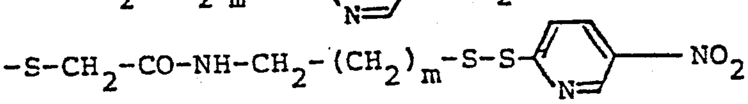

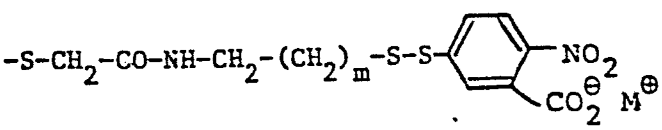

m étant défini comme précédemment ;

e) 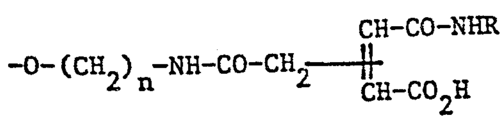

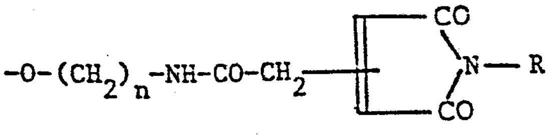

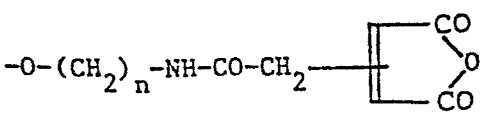

R étant défini comme précédemment et n étant un nombre entier variant de 1 à 6 ;

f) 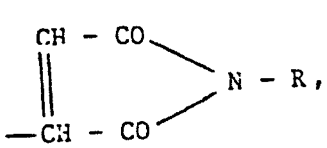 , 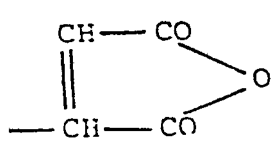 ou 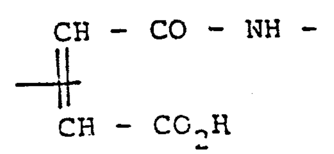

R étant défini comme précédemment.

7. Dérivés selon l'une quelconque des revendications 1 à 6, caractérisés par le fait que Y représente $Y_2$-$M_1$ ; $Y_2$ représentant le groupement résultant de la transformation du groupement fonctionnel $Y_1$ après couplage avec la molécule ($M_1$), et $M_1$ représentant le reste dérivé de la molécule ($M_1$) après couplage.

8. Dérivés selon la revendication 7, caractérisés par le fait que ($M_1$) est choisi parmi des antibiotiques, des peptides, des antimétabolites, des toxines, des enzymes, des dérivés fluorescents, des molécules susceptibles d'être couplées à des agents traceurs radioactifs, des molécules ou macromolécules aminées pouvant servir de sites réactifs en chromatographie d'affinité biospécifique, chimique ou physico-chimique, des molécules susceptibles de donner lieu à des interactions réversibles de type biospécifique, chimique ou physico-chimique.

9. Dérivés selon la revendication 8, caractérisés par le fait que la molécule ou macromolécule aminée ($M_1$) est choisie parmi les globulines, les toxines bactériennes, les lectines, les lectines succinylées, les antigènes bactériens, les antigènes viraux, les gangliosides ou leurs dérivés, la lysine, la taurine, la phénylalanine et la phénylhydrazine.

10. Dérivés selon la revendication 1, caractérisés par le fait qu'ils sont choisis parmi les composés suivants :

- β-D-ribofuranyl-thio-1-éthanoate de méthyle ;
- acide β-D-ribofuranyl-thio-1-éthanoïque ;
- β-D-ribofuranyl-thio-éthanoate d'hydroxysuccinimide ;
- 1-p-nitrobenzylthio-β-D-ribofuranide ;
- 1-p-aminobenzylthio-β-D-ribofuranide ;
- p-isothiocyanate de β-D-ribofuranyl-thiobenzyle :
- 1-p-diazobenzylthio-β-D-ribofuranide ;
- aminoéthyl-β-D-ribofuranoside ;
- β-ribofuranylthio-éthanoyl-aminoéthyl-4-phénol ;
- 4-β-ribofuranyl thioéthanoyl aminoéthyl-2,6-di-iodophénol ;
- O-(4-hydroxyphényl-propionamido-éthyl) β-D-ribofuranoside ;
- O(3,5-diiodo-4-hydroxyphényl) propionamido-éthyl-β-D-ribofuranoside ;
- ribofuranyl-thio-éthanoyl-fluorescine ;
- ribofuranyl-thio-éthanoyl phénylalanine ;
- ribofuranyl-thio-éthanoyl daunomycine ;
- ribofuranyl-thio-éthanoyl puromycine ;
- le produit de conjugaison de l'acide ribofuranyl-thio-éthanoïque et d'une peroxydase, d'une lectine ou d'une lectine succinylée ;

- le produit de formule

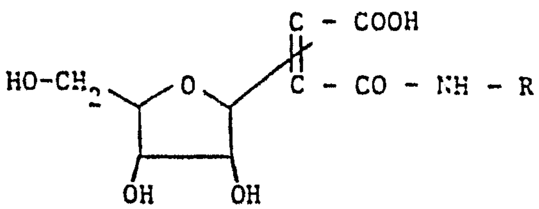

R étant défini comme précédemment;

- le produit de formule

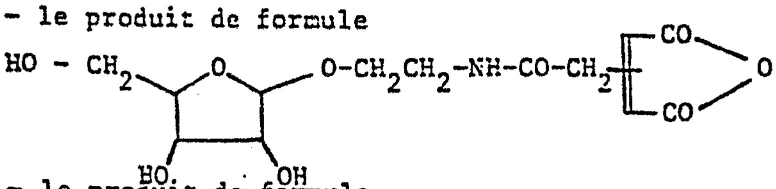

- le produit de formule

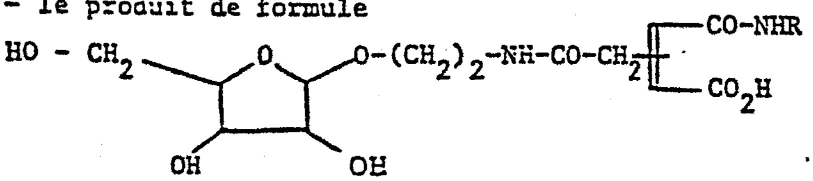

R étant défini comme précédemment ;

- l'anhydride C-ribofuranylmaléique.

11. Application des dérivés selon l'une quelconque des revendications précédentes, caractérisée par le fait que, dans le cas où $Y = Y_1$, l'on fait réagir le dérivé de formule I avec une molécule ($M_1$) capable de réagir avec le groupement réactif -Y pour former un dérivé de formule II

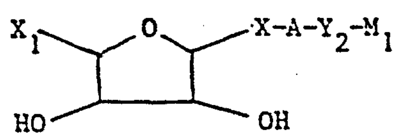

(II)

dans laquelle $X_1$, X et A sont définis comme dans la formule I, $Y_2$ représente le groupement résultant de la transformation du groupement fonctionnel $Y_1$ après couplage avec la molécule ($M_1$), et $M_1$ représente le reste dérivé de la molécule ($M_1$) après couplage, que l'on soumet, si désiré, le dérivé de formule II obtenu à une réaction de coupure oxydante pour former un dialdéhyde correspondant de formule III ou III A

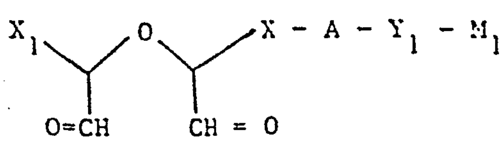

(III)

ou

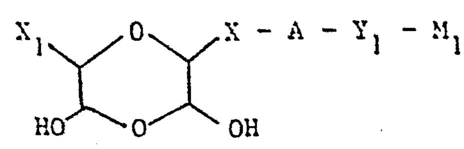

(IIIA) (forme dihydrate)

que l'on fait réagir avec une molécule ou macromolécule aminée $R_2NH_2$, pour donner un composé de formule IV

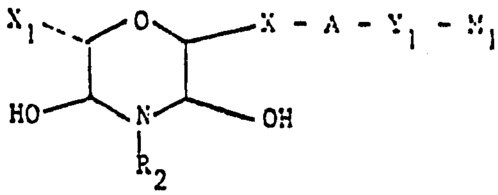

(IV)

que l'on réduit, si désiré, en un dérivé correspondant de formule V

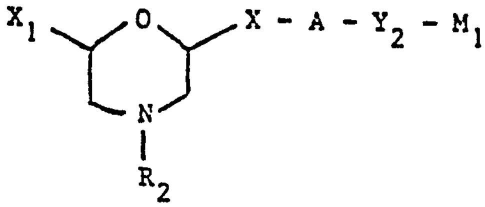

(V)

dans laquelle X, $X_1$, A, $Y_2$ et $M_1$ sont définis comme dans la formule II, et $R_2$ représente le reste de ladite molécule ou macromolécule aminée lorsque celle-ci est écrite sous la forme $R_2-NH_2$.

12. Application selon la revendication précédente, caractérisée par le fait que la molécule ou macromolécule aminée est choisie parmi un anticorps, une toxine, un fragment d'anticorps, un polymère aminé, ladite molécule ($M_2$) étant, si désiré, fixée sur un support solide.